# EUROPÄISCHE PATENTANMELDUNG

(11) **EP 0 820 771 A2**
(43) Veröffentlichungstag der Anmeldung: **28.01.1998**
(21) Anmeldenummer: 97112166.0
(22) Anmeldetag: 16.07.1997
(51) Int. Cl.: A61K 33/06, A61K 31/675, A61K 31/51, A61K 31/505

(54) **Arzneimittel zur Behandlung von Neuropathien, das ein lipidlösliches Thiamin und eine Magnesiumverbindung enthält**

(30) Priorität: 24.07.1996 DE 19629802
(71) Anmelder: WÖRWAG PHARMA GmbH, 71034 Böblingen (DE)
(72) Erfinder: Wörwag, Fritz, Dr., 70192 Stuttgart (DE)
(74) Vertreter: Weller, Wolfgang, Dr.rer.nat.

(57) **Zusammenfassung**

Ein Arzneimittel zur Prophylaxe und Therapie von Neuropathien weist als Wirkstoffe zumindest ein lipidlösliches Thiamin und einen Gehalt an zumindest einer Magnesiumverbindung auf.

## Beschreibung

Die Erfindung betrifft Arzneimittel, die zumindest ein lipidlösliches Thiaminderivat enthalten, zur Behandlung von Neuropathien.

Ein derartiges Arzneimittel ist aus der DE 42 06 422 A1 bekannt.

Die lipidlöslichen Thiaminderivate werden zusammen mit den wasserlöslichen Thiaminen unter dem allgemeinen Begriff Vitamin B1 zusammengefaßt. Als Vitamin ist Thiamin ein lebensnotwendiger Wirkstoff, der vom Organismus nicht selbst synthetisiert werden kann und deshalb über den Nahrungsweg aufgenommen werden muß. Die Thiamine sind vor allem für den Kohlenhydratstoffwechsel unentbehrlich. Die über die Nahrung aufgenommenen Thiamine werden im Körper in die biologisch wirksame Form, das Thiaminpyrophosphat (TPP), umgewandelt. Dabei ist das TPP ein essentieller Bestandteil von Enzymen, die an der Gewinnung von Energie und Stoffwechselswischenprodukten aus Kohlenhydraten beteiligt sind. Weder die über die Nahrung aufgenommenen Thiamine noch die biologisch aktive Form können vom Organismus gespeichert werden.

Aus der oben genannten DE 42 06 422 A1 geht hervor, daß Thiamine zur Behandlung von Neuropathien eingesetzt werden können. Neuropathien sind Nervenschädigungen, die in verschiedenen Formen vorkommen, bspw. als degenerative, toxische, metabolische oder ischämische Neuropathien, wobei die zuletzt genannte Form durch Durchblutungsstörungen zustande kommt. Unter dem Sammelbegriff Polyneuropathie werden nicht-entzündliche, degenerative Erkrankungen mehrerer peripherer Nerven verstanden, deren Ursachen hauptsächlich chronischer Alkoholismus, Diabetes mellitus oder Schwermetallvergiftungen sind.

Bei der Therapie und Prophylaxe von Neuropathien wirken sich vor allem die lipidlöslichen (d.h. fettlöslichen) Thiamine, insbesondere die Allithiamine, günstig aus. Das chemische Grundgerüst der wasserlöslichen Thiamine weist einen positiv geladenen Thiazoliumring auf. Die lipidlöslichen Allithiamine unterscheiden sich strukturell grundsätzlich dadurch, daß sie in einer offenen Thiolform vorliegen. Dabei ist die Thiolgruppe mit einem apolaren Rest modifiziert, der entweder aliphatisch oder aromatisch sein kann.

Die besondere Bedeutung der Thiamine für den Stoffwechsel des Nervensystems liegt darin begründet, daß im Nervensystem ausschließlich Kohlenhydrate, nicht aber Fette oder Proteine als Energiequelle herangezogen werden können. Dies zeigt sich besonders drastisch bei der Thiaminmangelkrankheit Beriberi (Polyneuritis), bei der vor allem das periphere Nervensystem betroffen ist. Diese Krankheit äußert sich in Bewegungsstörungen bis hin zur Paralyse, die durch Nervenschädigungen vor allem in den Extremitäten zustande kommen.

Aus dem Derwent Abstract 95 032 E/44 ist ein Entgiftungsverfahren zur Behandlung von Drogenabhängigen und Alkoholikern bekannt, bei dem unter anderem Magnesiumchlorid und Thiaminhydrochlorid (Vitamin B1) verabreicht werden. Das Vitamin B1 soll dabei dazu dienen, Neuritissymptome zu verhindern.

Aus dem Derwent Abstract 91-203 631/28 ist ferner ein Vitaminpräparat bekannt, in dem unter anderem Benfotiamin sowie Magnesiumaspartat enthalten sind.

In CHEMICAL ABSTRACTS 121: 308308z ist eine pharmazeutische Zusammensetzung beschrieben, die zur Behandlung oder Verhinderung von Umweltschädigungen eingesetzt werden soll, und die unter anderem Magnesiumcitrat und Vitamin B1 enthält. Außerdem ist es aus der EP 291 751 A2 bekannt, ein Hydroxiphenylalkanolamin zusammen mit Vitamin B1 und einem Magnesiumsalz zur Behandlung von Heuschnupfen einzusetzen.

Bei der Therapie von Neuropathien mit lipidlöslichen Thiaminen wurde festgestellt, daß es einen bestimmten Prozentsatz von Patienten gibt, die auf eine solche Therapie nicht ansprechen (sogenannte "Non-Responser"). Auch eine hochdosierte Verabreichung der lipidlöslichen Thiamine führte nicht dazu, daß die Non-Responser auf die Therapie ansprachen.

Es ist nun Aufgabe der vorliegenden Erfindung, ein Arzneimittel zu schaffen, mit dessen Hilfe auch Patienten, die auf die Behandlung mit lipidlöslichen Thiaminen alleine nicht ansprechen, therapiert werden können.

Erfindungsgemäß wird die Aufgabe dadurch gelöst, daß als Wirkstoffe Benfotiamin und Magnesiumorotat enthalten sind.

Hierbei ist von Vorteil, daß einerseits Benfotiamin eine geringe Toxizität aufweist und zudem die Bluthirnschranke passieren kann und daß andererseits die Orotsäure als Fixateur des Magnesiums in der Zelle wirkt.

Bei einer Verabreichung von Benfotiamin und Magnesiumorotat haben auch Non-Responser auf die Behandung angesprochen.

Darüber hinaus zeigte sich ein überraschender und langanhaltender Effekt bei denjenigen Patienten, die auf die Behandlung mit lipidlöslichen Thiaminen alleine nicht ansprachen, wie dies anhand einer weiter unten ausgeführten Studie belegt werden konnte.

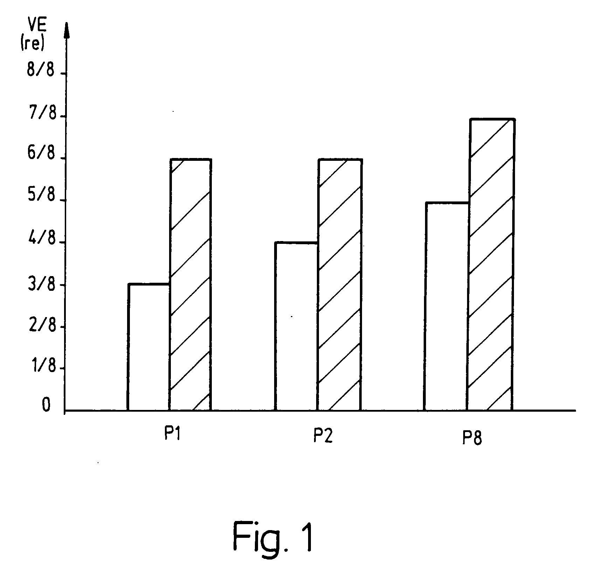

Es war zwar in Untersuchungen von Itokawa et al. festgestellt worden, daß die Bioverfügbarkeit von wasserlöslichen Thiaminen bei Magnesiummangel sehr niedrig ist, jedoch wurde dieser Effekt hauptsächlich in der Leber, im Herzen und in den Nieren nachgewiesen, nicht aber im Nervensystem (Y. Itokawa und M. Kimura, "Effect of magnesium deficiency on thiamin metabolism", Magnesium-Bulletin 1/1982, Seite 5-8, Fig. 1 auf Seite 6, Fig. 4 auf Seite 7, Fig. 5 auf Seite 8). Das bedeutet, daß die Bioverfügbarkeit von wasserlöslichem Thiamin im Nervensystem offenbar nicht prägnant vom Magnesiumstatus abhängig ist. Daher ist es umso überraschender, daß die Verabreichung eines lipidlöslichen Thiamins und einer Magnesiumverbindung bei der Bekämpfung eines Nervenleidens wie der Neuropathie erfolgreich eingesetzt werden kann.

Dabei kann das erfindungsgemäße Arzneimittel nicht nur bei der Therapie von Non-Responsern zur Anwendung gebracht werden. Auch bei der Behandlung von solchen Patienten, die bereits auf die Therapie mit einem lipidlöslichen Thiamin alleine ansprechen, kann es dazu eingesetzt werden, den Therapieerfolg zu verbessern.

Die Erfindung betrifft deshalb auch die Verwendung eines Arzneimittels, das zumindest ein lipidlösliches Thiamin sowie einen zusätzlichen Gehalt an zumindest einer Magnesiumverbindung zur Behandlung von Neuropathien aufweist.

Außerdem betrifft die Erfindung die Verwendung eines Arzneimittels, das zumindest ein lipidlösliches Thiamin sowie einen zusatzlichen Gehalt an zumindest einer Magnesiumverbindung aufweist, zur Behandlung von Polyneuropathien sowie von Neuropathien, die im Zusammenhang mit Alkoholmißbrauch, Mangelernährung, Stoffwechselerkrankungen oder toxischen Stoffen stehen.

Wie in der weiter unten vorgestellten Studie belegt wird, wirkt sich das erfindungsgemäße Arzneimittel besonders vorteilhaft bei der Prophylaxe und Therapie diabetischer Neuropathien aus. Eine besondere Bedeutung ist hier der Prophylaxe beizumessen, denn die mit der Neuropathie einhergehenden biochemischen Stoffwechselveränderungen der Nervenzellen sind nur noch in einem frühen Stadium beeinflußbar und teilweise reversibel, in späteren Stadien kommt es dagegen zu irreversiblen morphologischen Veränderungen mit schwerwiegenden Komplikationen.

Somit wird die Aufgabe vollkommen gelöst.

In einer bevorzugten Ausgestaltung der Erfindung werden unter den lipidlöslichen Thiaminderivaten die Allithiamine ausgewählt, die nach oraler Einnahme erheblich besser intestinal resorbiert werden als die wasserlöslichen Thiamine (R. Bitsch und I. Bitsch, "Lipidlösliche Thiaminderivate", Deutsche Apothekerzeitung, Nr. 2, 1989, Seiten 65-68). Daher werden bei oraler Verabreichung von Allithiaminen wesentlich höhere Thiaminspiegel in Blut und Gewebe von Organismen erreicht als bei der Gabe gleicher Mengen wasserlöslicher Thiamine. Außerdem werden Allithiamine wesentlich länger im Körper zurückgehalten, d.h. in weniger großen Mengen ausgeschieden als andere Thiaminderivate.

Unter den Allithiaminen sind die aromatischen Derivate wie das Benfotiamin, Bentiamin und Octotiamin sowie das nicht aromatische Fursultiamin zu bevorzugen, da sie im Gegensatz zu den aliphatischen Vertretern nicht geruchsintensiv sind und zudem eine höhere Hitzestabilität und eine vermehrte Resistenz gegenüber Thiaminasen aufweisen. Außerdem weisen die Allithiamine gegenüber dem Thiaminchloridhydrochlorid, einem löslichen Thiamin, eine vergleichsweise geringere Toxizität auf und sind so therapeutisch besser einsetzbar.

Als Magnesiumverbindungen werden bevorzugt Magnesiumorotat, Magnesiumaspartat, Magnesiumhydrogenaspartat, Magnesiumglukonat, Magnesiumhydrogenphosphat, Magnesiumcitrat, Magnesiumhydrogencitrat, Magnesiumchlorid, Magnesiumhydrochlorid, Magnesiumcarbonat, Magnesiumacetat, Magnesiumbenzoat, Magnesiumlactat, Magnesiumhydrogenglutamat, Magnesiumoxid oder Magnesiumsulfat eingesetzt.

In einer höchst vorteilhaften Ausgestaltung der Erfindung wird ein Arzneimittel zur Behandlung von Neuropathien eingesetzt, das als Wirkstoff Benfotiamin und Magnesiumorotat enthält. Hier liegt als erster Wirkstoff das günstig resorbierbare, lange im Körper verbleibende und wenig toxische Allithiamin Benfotiamin vor und als zweiter Wirkstoff die Magnesiumverbindung Magnesiumorotat, die besonders effizient von Zellen aufgenommen werden kann.

Es versteht sich, daß die vorstehend genannten und die nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

Die Erfindung wird nachfolgend anhand einer Studie mit insgesamt neun Patienten, die an schmerzhaften Neuropathien in Füßen und Beinen litten, näher erläutert. Die Patienten wurden entweder zunächst für vier bis acht Wochen mit Benfotiamin alleine therapiert und danach mit Benfotiamin und zusätzlich einer Magnesiumverbindung, oder sie wurden direkt mit Benfotiamin und einer Magnesiumverbindung behandelt.

### Patient 1

Ein 61-jähriger Patient, der seit elf Jahren an Typ II-Diabetes mellitus leidet, litt für sechs Monate am sogenannten Burning-Feet-Syndrom (einem Brennen der Füße, das anfallsweise und meist nachts auftritt) sowie an subjektiv sehr unangenehmen Sensibilitätsstörungen, bei denen jede Berührung als Schmerz empfunden wird. Nachdem die Gabe von täglich 4 x 80 mg an Benfotiamin alleine über einen Zeitraum von sechs Wochen keine wesentliche Besserung der Beschwerden hervorgebracht hatte, wurden zusätzlich täglich 2 x 1 g an Magnesiumorotat verabreicht. Bereits nach zehn Tagen hatten sich die Sensibilitätsstörungen dieses Patienten erheblich verbessert.

Dieser Patient, der auf die Therapie mit Benfotiamin alleine nicht ansprach (Non-Responser), wurde durch die zusätzliche Gabe von Magnesiumorotat zum Responser.

### Patient 2

Ein 36-jähriger Patient, der seit 16 Jahren an Typ I-Diabetes mellitus leidet, war für mehr als zwei Jahre zusätzlich an einer Neuropathie erkrankt, insbesondere einem Taubheitsgefühl in beiden Füßen, beginnend auch in den Unterschenkeln, sowie an einer zunehmenden Gangunsicherheit. Er wurde zunächst für vier Wochen mit 3 x 80 mg an Benfotiamin therapiert. Danach wurden zusätzlich 3 x täglich 500 mg an Magnesiumorotat verabreicht.

Unter dieser Therapie wurde nach ca. vier Wochen ein Rückgang des Taubheitsgefühls in den Unterschenkeln sowie eine Verbesserung des Taubheitsgefühls in den Füßen beobachtet.

Auch dieser auf die Therapie mit Benfotiamin alleine nicht ansprechende Patient wurde mit Hilfe des erfindungsgemäßen Arzneimittels zum Responser.

### Patient 3

Ein 57-jähriger Patient, der seit acht Jahren an Typ II-Diabetes mellitus leidet, litt seit eineinhalb Jahren zusätzlich an einem Taubheitsgefühl, Kribbeln und Eingeschlafensein beider Füße, sowie seit zwei Monaten an einem Brennen der Füße. Nachdem sich bei einer Therapie mit täglich 3 x 80 mg an Benfotiamin über einen Zeitraum von acht Wochen keine Besserung der Beschwerden eingestellt hatte, wurden daraufhin zusätzlich 3 x täglich 500 mg an Magnesiumorotat verabreicht. Innerhalb eines zwölfwöchigen Beobachtungszeitraums war eine deutliche Besserung der subjektiven Beschwerden zu verzeichnen.

Auch dieser mit Benfotiamin alleine nicht therapierbare Patient konnte durch die Behandlung mit Benfotiamin und Magnesiumorotat erfolgreich behandelt werden.

### Patient 4

Ein seit 10 Jahren an Typ I-Diabetes mellitus leidender, 33-jähriger Patient litt seit eineinhalb Jahren an einer zunehmenden, kribbelnden Empfindungsstörung in beiden Füßen. Dazu kamen eine leichte Gangunsicherheit sowie nächtliche Schmerzen in beiden Unterschenkeln. Nach einer täglichen Therapie mit 3 x 80 mg an Benfotiamin sowie 2 x täglich 120 mg an Magnesiumhydrogenaspartat über einen Zeitraum von acht Wochen hinweg, zeigte sich ein deutlicher Rückgang der nächtlichen Wadenschmerzen, eine leichtgradige Verbesserung des Kribbelns sowie eine subjektive Verbesserung der Gangunsicherheit. Auch nach drei Monaten ohne Therapie war keine Wiederverschlechterung der Beschwerden zu verzeichnen.

Die neuropathischen Beschwerden dieses Patienten konnten also mit Hilfe des erfindungsgemäßen Arzneimittels erfolgreich bekämpft werden. Darüber hinaus zeigte sich bei diesem Patienten auch nach Absetzung des Arzneimittels eine langanhaltende Wirkung.

### Patient 5

Ein 57-jähriger Patient, der seit vier Jahren an Typ II B-Diabetes mellitus leidet, klagte über Brennen in beiden Füßen sowie ausgeprägte, besonders nachts auftretende stechende Schmerzen in beiden Unterschenkeln seit mehreren Monaten. Nach Therapie mit 4 x täglich 80 mg an Benfotiamin sowie 2 x täglich 50 mg Magnesium (in Form von Magnesiumhydrogenphosphat und Magnesiumcitrat) war ein deutliches Nachlassen der stechenden Schmerzen und eine Besserung des Brennens zu verzeichnen.

Auch hier zeigte die Therapie mit Benfotiamin und einer Magnesiumverbindung einen deutlichen therapeutischen Erfolg.

### Patient 6

Eine seit 17 Jahren an Typ II B-Diabetes mellitus leidende, 73-jährige Patientin klagte über seit zwei Jahren auftretende Taubheitsgefühle in beiden Füßen, stechende Schmerzen in beiden Unterschenkeln sowie eine Gangunsicherheit. Unter einer Therapie mit täglich 4 x 80 mg an Benfotiamin sowie 3 x täglich 120 mg an Magnesiumhydrogenaspartat verbesserten sich die stechenden Schmerzen und die Gangunsicherheit mäßig.

Auch bei dieser Patientin war mit Hilfe des erfindungsgemäßen Arzneimittels eine Besserung der Beschwerden zu verzeichnen.

### Patient 7

Ein seit sieben Jahren an Typ II-Diabetes mellitus leidender 63-jähriger Patient klagte über zunehmende nächtliche Wadenkrämpfe, ein Brennen beider Füße sowie eine Gangunsicherheit seit sechs Monaten. Nach einer dreimonatigen Therapie mit 3 x täglich 80 mg an Benfotiamin sowie 3 x täglich 120 mg an Magnesiumhydrogenaspartat war eine deutliche Besserung der stechenden Schmerzen zu verzeichnen.

Auch hier zeigte das Arzneimittel bestehend aus Benfotiamin und einer Magnesiumverbindung einen wesentlichen therapeutischen Erfolg.

### Patient 8

Ein 43-jähriger Patient, der seit zwölf Jahren an Typ I-Diabetes mellitus leidet, klagte über zunehmende Sensibilitätsstörungen, brennende Schmerzen in beiden Füßen und nächtliche Wadenkrämpfe seit einem Jahr. Nach einer Therapie mit 3 x täglich 80 mg an Benfotiamin und 3 x täglich 500 mg an Magnesiumorotat verbesserten sich nach acht Wochen deutlich die subjektiven Beschwerden, und die nächtlichen Wadenkrämpfe blieben aus. Nach Absetzen der Medikation war über einen Beobachtungszeitraum von sechs Monaten hinweg keine signifikante Wiederzunahme der Beschwerden zu verzeichnen.

Die neuropathischen Beschwerden dieses Patienten waren also mit Hilfe des erfindungsgemäßen Arzneimittels erfolgreich bekämpft worden. Darüber hinaus wies das Arzneimittel eine über den Zeitraum von mindestens einem halben Jahr andauernde Wirkung auf.

### Analyse des Vibrationsempfindens

Das Vibrationsempfinden wird durch Aufsetzen einer schwingenden, skalierten Stimmgabel auf oberflächlich liegende Skeletteile geprüft. Mit Hilfe dieses sogenannten Riedel-Seyfert-Tests erhält man einen objektivierbaren neurologischen Parameter, der Auskunft über das Ausmaß der beim Patienten vorliegenden Sensibilitätsstörungen gibt.

Drei der Patienten der oben ausgeführten Studie, die Patienten 1, 2 und 8, wurden diesem Test unterworfen. Die daraus erhaltenen Ergebnisse sind in der beiliegenden Zeichnung graphisch dargestellt.

Die einzig beiliegende Zeichnung zeigt ein Balkendiagramm, das die Verbesserung des Vibrationsempfindens (VE) der drei Patienten vor und nach Behandlung mit dem erfindungsgemäßen Arzneimittel aufzeigt.

Den Patienten 1 und 2 war zunächst für sechs bzw. vier Wochen Benfotiamin alleine verabreicht worden. Patient 8 war zunächst nicht mit Benfotiamin therapiert worden. Danach wurde allen drei Patienten täglich 3 x 80 mg Benfotiamin und Magnesiumorotat in einem Verhältnis von Benfotiamin zu Magnesiumorotat von 1:17 verabreicht. Das Vibrationsempfinden der Patienten wurde vor Beginn der Therapie mit Benfotiamin und Magnesiumorotat gemessen, was in dem Diagramm durch einen weißen Balken aufgezeigt wird. Nach der Therapie mit Benfotiamin und Magnesiumorotat für zwei Wochen (Patient 1), vier Wochen (Patient 2) bzw. acht Wochen (Patient 8) wurde das Vibrationsempfinden erneut gemessen. Die Ergebnisse dieser Messungen sind in dem Diagramm durch einen schraffierten Balken dargestellt. Im vorliegenden Diagramm sind die Werte aufgezeigt, die für das Vibrationsempfinden am rechten großen Zeh (re) erhalten wurden, wobei das Vibrationsempfinden auf einer Skala von 1/8 bis 8/8 gemessen wird. Während sich das Vibrationsempfinden des Patienten 1 durch die kombinierte Gabe von Benfotiamin und Magnesiumorotat von 3/8 auf 6/8 verbesserte, steigerte sich das Vibrationsempfinden des Patienten 2 von 4/8 auf 6/8 und des Patienten 8 von 5/8 auf 7/8.

Somit war bei allen Patienten ein erhöhtes Vibrationsempfinden nachweisbar, das sich um 30 bis 50 % gegenüber dem Ausgangswert steigerte. Daraus ergibt sich, daß die Sensibilitätsstörungen aller drei Patienten mit Hilfe des erfindungsgemaßen Arzneimittels erfolgreich therapiert werden konnten.

Aus der hier vorgestellten Studie mit insgesamt acht Patienten mit neuropathischen Beschwerden ergibt sich zusammenfassend, daß bei denjenigen Patienten, die auf eine Therapie mit Benfotiamin alleine nicht ansprechen, eine wesentliche und zudem teilweise langandauernde Verbesserung der Beschwerden durch eine Therapie mit Benfotiamin und einer Magnesiumverbindung erreicht werden kann. Dies drückt sich in einem verbesserten Vibrationsempfinden der Extremitäten, dem Nachlassen stechender Schmerzen oder nächtlicher Krämpfe, sowie der erfolgreichen Bekämpfung schmershafter Sensibilitätsstörungen und Taubheitsgefühlen in den Beinen und Füßen aus.

## Patentansprüche

1. Arzneimittel, dadurch gekennzeichnet, daß Benfotiamin und Magnesiumorotat enthalten ist.

2. Verwendung eines Stoffgemischs, das zumindest ein lipidlösliches Thiamin sowie einen zusätzlichen Gehalt an zumindest einer Magnesiumverbindung enthält, zur Herstellung eines Arzneimittels zur Behandlung von Neuropathien.

3. Verwendung eines Stoffgemischs, das zumindest ein lipidlösliches Thiamin sowie einen zusätzlichen Gehalt an zumindest einer Magnesiumverbindung enthält, zur Herstellung eines Arzneimittels zur Behandlung von Neuropathien sowie von Neuropathien, die im Zusammenhang mit Alkoholmißbrauch, Mangelernährung, Stoffwechselerkrankungen oder toxischen Stoffen stehen.

4. Verwendung eines Stoffgemischs nach Anspruch 2 oder 3, dadurch gekennzeichnet, daß als lipidlösliches Thiamin ein Allithiamin verwendet wird.

5. Verwendung eines Stoffgemischs nach einem der Ansprüche 2 bis 4, dadurch gekennzeichnet, daß als Allithiamin Benfotiamin, Fursultiamin, Octotiamin oder Bentiamin verwendet wird.

6. Verwendung eines Stoffgemischs nach einem der Ansprüche 2 bis 5, dadurch gekennzeichnet, daß als Magnesiumverbindung Magnesiumorotat, Magnesiumaspartat, Magnesiumhydrogenaspartat, Magnesiumglukonat, Magnesiumhydrogenphosphat, Magnesiumchlorid, Magnesiumhydrogenchlorid, Magnesiumcitrat, Magnesiumhydrogencitrat, Magnesiumcarbonat, Magnesiumacetat, Magnesiumbenzoat, Magnesiumlactat, Magnesiumhydrogenglutamat, Magnesiumoxid oder Magnesiumsulfat verwendet wird.

7. Verwendung eines Stoffgemischs nach einem der Ansprüche 2 bis 6, dadurch gekennzeichnet, daß als Allithiamin Benfotiamin und als Magnesiumverbindung Magnesiumorotat verwendet wird.
